# EUROPEAN PATENT APPLICATION

(11) **EP 2 918 291 A2**
(43) Date of publication of application: **16.09.2015**
(21) Application number: 15158192.3
(22) Date of filing: 09.03.2015
(51) Int. Cl.: A61K 47/02, A61K 47/12, A61K 47/18, A61K 47/26, A61K 47/36, A61K 47/40

(54) **PHARMACEUTICAL COMPOSITION OF STABILIZED PARACETAMOL, PLASTIC BAGS AND USE OF THE PHARMACEUTICAL COMPOSITION**

(30) Priority: 13.03.2014 BR 102014005885
(71) Applicant: Bio Biologica Industria Farmaceutica Ltda., 74.703-100 Goiania - Estado de Goias (BR)
(72) Inventor: Badauy Lauria Silva, Gisele, 74.703-100 Goiânia - Estado de Goiás (BR); Augusto Gonçalves, César, 74.703-100 Goiânia - Estado de Goiás (BR); Desideri, Viviane, 74.703-100 Goiânia - Estado de Goiás (BR)
(74) Representative: Pons Ariño, Angel

(57) **Abstract**

The present invention relates to a stabilized pharmaceutical composition paracetamol conveyed in plastic bag and ready for use by perfusion.

## Description

### Field of the Invention

The present invention relates to a stabilized pharmaceutical composition paracetamol conveyed in plastic bag and ready for use by perfusion.

### Background of the Invention

The compound paracetamol (N-acetyl-4-aminophenol, C₈H₉NO₂) has been widely used as active ingredient in pharmaceutical compositions due to its activity as an analgesic and antipyretic. The compound is represented by the following structural formula:

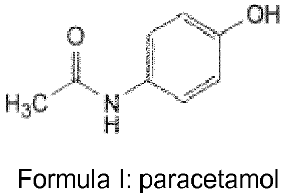

Moreover, the compound is well tolerated by human beings and does not alter the acid-base balance, reason why it has been used to combat pain, both in adults and in children or elderly.

Usually, pharmaceuticals products comprising paracetamol have been provided for administration orally or topically. This is because it is difficult to obtain a pharmaceutical preparation for injection and particularly, a ready solution to be used by perfusion due to the fact that paracetamol is not very soluble in water and its solutions in aqueous medium are unstable in the presence of oxygen and/or light, being decomposed through a plurality of degradation pathways which are well known and are described, for example, in the article "Stability of aqueous solutions of N-acetyl-p-aminophenol" by KT Koshy and JL Lach, J. Pharmaceutical Sciences, Vol. 50 (2) February 1961, p. 113-118. This instability in aqueous medium is detected by the appearance of substances causing the coloring of the solution, for example, benzoquinoneimine which are hepatotoxic in humans.

The development of color in pharmaceutical solutions, particularly injectables should be completely transparent, it becomes a serious problem, since the presence of said color is indicative of the existence of unwanted compounds in the composition and therefore leads to the rejection of the injectable product cannot be applied to the patient.

One of the causes of paracetamol degradation is based on chemical oxidation reactions in which the oxygen present in the solution is the main precursor of this degradation. The secondary cause of degradation may be the deacetylation of the amino group, generating p-aminophenol, which is also quickly degraded producing p-benzoquinoneimine. This deacetylation takes place both at acid pH (much faster) as well as in alkaline pH once the phenolate form is present.

*In vivo,* most of the paracetamol is metabolized through the formation of these phenolate form derivatives, mainly through the derivative gluconate and sulfonated derivatives.

Pharmaceutical compositions of stable paracetamol, as a solution in an aqueous medium, can be obtained through joint actions of: (1) establish an appropriate pH and (2) avoid the presence of oxygen in the solution.

The verification of an optimal pH in which the formation of 4-aminophenol is prevented or minimized, has been indicated by K. Thomas Koshy and Jon L. Lach in the article "Stability of aqueous solutions of N-acetyl-p-aminophenol", by KT Koshy and JL Lach, J. Pharmaceutical Sciences, Vol. 50 (2) February 1961, p. 113-118, where the hydrolysis of the acetate group of paracetamol is minimized between pH 4.5 and 6.0.

EP 858329 patent, Pharmatop SCR teaches to avoid the presence of oxygen in the paracetamol solution. This patent document discloses a process in which the paracetamol oxidation is prevented by means of eliminating the main reaction activation element (oxygen) by nitrogen bubbling. Keeping the solution obtained in a completely hermetic bottle (glass vial), the stability of paracetamol in solution is ensured for long time periods, with minimal impurity levels and the total absence of color in the solution. The combined action of the two previous factors allows obtaining a stable paracetamol solution which does not develop color for a long period of time.

The international patent application WO2004/071502, Nguyen-Xuan, describes a paracetamol formulation containing a buffer agent with a pKa between 4.5 and 6.5, an isotonic agent and a paracetamol dimer. The stability of paracetamol in solution is attributed to the presence of the paracetamol dimer produced *in situ* by treatment of the solution at a temperature between 100°C and 130°C for at least 5 minutes. Because it is not necessary to eliminate oxygen, the solution may be stored in some plastic materials. However, it has the following drawbacks:
(1) it does not contain factors preventing the oxidation of paracetamol, generating impurities over time, as the mentioned formation of dimer, providing color to the solution and turning it into a product that is not safe to use because, at the time of its use, it is not possible to know if the color comes from the paracetamol dimer formation, benzoquinoneimines or other substances with unknown origin.
(2) The stability of these solutions is reduced when they are stored in plastic materials such as PVC, whose composition does not use antioxidants. That is, such state of the art compositions should be stored in plastic materials such as polypropylene, polyolefins, polyethylene, polyethylene vinyl acetate, containing antioxidants or are capable of preventing the entry of oxygen into the solution. These materials contain one of the following antioxidants:
   1. Butyl hydroxy toluene
   2. pentaerythrityl tetrakis (3,5-di-tert-butyl-4-hydroxyphenyl) propionate;
   3. 1,3,5-tris (3,5-di-tert-butyl-4-hydroxybenzyl) -s-triazine-2,4,6(1 H, 3H, 5H)-trione;
   4. octadecyl 3-(3,5-di-tert-butyl-4-hydroxyphenyl)propionate;
   5. ethylenebis[3,3-bis[3-(1,1-dimethylethyl)-4-hydroxyphenyl]butanoate];
   6. dioctadecyl disulfide;
   7. 2,2', 2', 6,6', 6"-hexa-tert-butyl-4,4', 4"- [(2,4,6-trimethyl-1,3,5-benzenotriil)trimethylene]triphenol;
   8. 2,2'-bis(octadecyloxy)-5,5'-spirobi[1,3,2-dioxafosfinano];
   9. didodecyl 3,3'-thiodipropionate;
   10. dioctadecyl 3,3'-thiodipropionate;
   11. tris(2,4-di-tert-butylphenyl)phosphate;
   12. Five different substances containing phenyl fosfinita group.
   13. Butylhydroxyanisole

The patent document FR 2 751 875 discloses aqueous paracetamol solutions for use as a perfusion having a pH between 5 and 7. The pH value is maintained by a buffer system comprising an acid and an alkali metal acetate or phosphate. It further comprises stabilizing substances such as sulfurous acid group derivatives, for example, sulphoxilate formaldehyde. Patent document FR 2 809 619 discloses aqueous paracetamol solutions for use in perfusion, having pH between 5 and 6, preferably 5.5. The pH value is maintained by a buffer system, such as a mixture citric acid/trisodium citrate. It further comprises stabilizing substances such as sulfite or derivatives thereof in a concentration between 0.00001% and 0.1 % w/v. Patent US 6 028 222 discloses the use of aqueous paracetamol solution for use in perfusion comprising stabilizing substances such as glucose or mannitol polyols. However, no concentration range for glucose is taught. Therefore, there remains a need to develop a ready for use injectable stable paracetamol pharmaceutical composition, which is capable of preventing the development of unwanted color in the solution over time and can be stored in a plastic bag.

### Description of the Invention

In order to overcome the drawbacks of the state of the art, the present invention provides a ready for use injectable stable paracetamol pharmaceutical composition, which can be stored in a plastic bag and prevents the development of unwanted color in the solution over time.

The technical solution is based on the fact that substances capable of reacting with p-acetyl aminophenolate form, an intermediate chemical species in the degradation both by oxidation and by deacetylation were identified. When these paracetamol degradation pathways are significantly reduced, a highly stable injectable paracetamol solution with a minimal content of impurities can be obtained. The reason for this is that the formation of intermediates such as gluconate or sulfonated derivatives with the phenolate form, although they can be unstable in solution, they significantly reduce paracetamol degradation. Sulphate, gluconate or furfural ions can be found in solution as byproducts generated by this degradation. Consequently, pharmaceutical compositions or stable aqueous paracetamol solutions for their use by perfusion according to the present invention comprise at least one substance that can react with phenolates turning them into their O-derivatives or coordination compounds.

In a particular realization of the invention, the substance may be selected from reducing sugars such as glucose, galactose, fructose, mannitol, the acid forms of these sugars or their salts such as mannitol, gluconate, glucuronate, glucoheptanoate, galactato, chemical species containing sulfur in an oxidation state less than 6, sodium formaldehyde sulfoxylate, thiourea or sulfites or any combination of the previous substances. The use of these substances in the pharmaceutical composition according to the present invention can produce a solution with very reduced levels of impurities and the absence of color in the solution for long periods of time and may be stored in plastic materials even free of antioxidants.

It is possible to find aqueous paracetamol pharmaceutical compositions for perfusion containing antioxidants in the state of the art. However, the different antioxidant power of different substances through the reactivity of phenolate in aqueous solution or its possible practical consequences have never been studied.

We have now found that the action on this intermediate compound in the process of hydrolysis/oxidation of paracetamol allows providing different degrees of protection of paracetamol against oxidation in the same pH conditions, such that it is possible to obtain a solution with the above advantages mentioned choosing the appropriate antioxidant or substance that can react with the intermediate phenolate form.

A very important factor to be considered is that the balance must be achieved between solution color/paracetamol degradation impurities and impurities from the degradation of substances used as stabilizers. Although the prior art has described aqueous paracetamol solutions for perfusion comprising mannitol, glucose, fructose or gluconate as an isotonic agent, using amounts of about 5% w/v to provide isotonicity to the solution is required, with which a solution color values is obtained after a few months of its production and therefore this solution would not be suitable in the present invention.

Therefore, it is necessary to use the suitable amount for each stabilizing compound so that said compound exhibits its stabilizing effect without developing any substantial color over time. In the paracetamol solutions according to the invention, this is achieved by adding at least one stabilizing substance of paracetamol in solution selected from the group consisting of mannitol, glucose, fructose, or gluconate in a concentration between 0.4% w/v and 3.3% w/v and/or sodium formaldehyde sulfoxylate, sodium sulfite or sodium dithionite in a concentration between 0.0008% and 0.02% w/v.

The addition of the cited stabilizing substances is not intended to provide isotonicity to the solutions according to the present invention and moreover it would be insufficient for that purpose. On the contrary, their addition is intended to provide the technical effect described, i.e., to stabilize the paracetamol in solution, thereby delaying the development of a color in the solution with time. This effect is non-obvious and could not be expected from the description of the prior art.

A last aspect to be considered is that the solubility of paracetamol in aqueous medium is of the order of 12 mg/ml at a temperature of 20 °C and 8 mg/ml at 4 °C to prevent crystallization of paracetamol. This effect is achieved by filtering the solution through a pore size of 0.45 microns or less, or adding a solubilizing agent as described in international patent application WO03033026, filed by Bioren SA, which teaches a aqueous paracetamol solution obtained by mixing paracetamol and propylene glycol in citrate medium at a pH between 4.5 and 6.5 and heating said solution at a temperature between 70 °C -130 °C.

The concentration of antioxidant has an important role in the stabilization of the solution because the degradation of such antioxidant in turn generates impurities providing color to the solution. Sugar can produce other derivatives furfural gluconate and reducing substances of sulfur can produce sulfonated derivatives of paracetamol. These, in turn, can also provide color to the solution.

The concentration of the stabilizing substance can vary. In a particular realization of the invention, the pharmaceutical compositions according to the present invention comprise at least one stabilizing substance of paracetamol in solution, more particularly mannitol in a concentration from 0.2% to 3.5% w/v, especially a concentration from 0.6% to 3.3% w/v.

In another embodiment, the stabilizing substance of paracetamol in solution is cysteine hydrochloride monohydrate as a free radical collector at a concentration from 0.01% to 1% w/v, particularly at a concentration between 0.01 % to 0.5 % w/v.

In another embodiment, the stabilizing substance of paracetamol in solution is anhydrous dibasic sodium phosphate as buffer for stabilizing the pH range from 5.5 to 5.7 at a concentration between 0.01% to 1% w/v.

In another embodiment, the stabilizing substance of paracetamol in solution is hydroxyethyl starch (HES1) as a solubilizing aid and a stabilizer in a concentration between 0.1% to 2% w/v, particularly at a concentration between 0.1 % to 1 5% w/v.

In another realization, the stabilizing substance of paracetamol in solution is hydroxypropyl gamma-cyclodextrin as a solubilizing aid and a stabilizer in a concentration between 0.1% to 2% w/v, particularly at a concentration between 0.1 % to 1 5% w/v.

In another embodiment, the stabilizing substance of paracetamol in solution is sodium acetate trihydrate as a buffering agent to stabilize the pH from 5.5 to 5.7 at a concentration between 0.1% to 1% w/v, particularly at a concentration between 0.1 % to 0.5% w/v.

In another embodiment, the stabilizing substance of paracetamol in solution is sodium citrate dihydrate as a buffering agent to stabilize the pH from 5.5 to 5.7 at a concentration between 0.1 % to 1% w/v.

The vehicle of the present invention is water for injectable, particularly qs 1 mL.

The pharmaceutical compositions according to the present invention may further comprise one or more substances for adjusting the pH from 5.5 to 5.7, 1 M solution, selected from sodium hydroxide, glacial acetic acid, hydrochloric acid.

The pharmaceutical compositions according to the present invention may further comprise one or more osmolarity stabilizing substance, which can be glucose, galactose, fructose or mannitol, to obtain an isotonic solution with osmolarity of 260 mOsm to 320 mOsm, in particular in a concentration of 0.5% to 5%, particularly 3.3%.

The pharmaceutical composition according to the present invention is suitable for use in a plastic bag. In particular embodiment, primary container in polypropylene (PP) tri-laminate. The bag ready for use may have 50 ml with a concentration of 500 mg/50 ml, 100 ml with a concentration 1000 mg/100 ml, 125 ml with a concentration 1250 mg/125 ml.

In particular embodiment, use is made of a secondary packaging of cardboard cartridge, which protects the bag from direct light.

The following examples serve to illustrate aspects of the present invention without having, however, any limiting character.

### Example 1

**Table 1 - pharmaceutical compositions according to the present invention**

| Raw material | F1 | F2 | F3 | F4 | F5 | F6 |
|---|---|---|---|---|---|---|
| Paracetamol | 1% | 1% | 1% | 1% | 1% | 1% |
| Mannitol Injection | 3.1% | 3.1% | 3.85% | 3.1% | 3.1% | 3.1% |
| Hydroxyethyl starch (HES1) | 1% | 1% | - | 0.5% | 1% | 1% |
| Sodium acetate trihydrate | 0.3% | 0.3% | - | - | 0.3% | 0.3% |
| Sodium Citrate Dihydrate | 0.3% | 0.3% | - | - | 0.3% | 0.3% |
| Acetic acid solution 10% q.s. pH 5.5 | q.s | q.s | - | - | q.s | - |
| Cysteine Hydrochloride Monohydrate | - | - | 0.025% | 0.025% | 0.025% | 0.025% |
| Disodium Phosphate Dibasic Anhydrous | - | - | 0.014% | 0.014% | 0.014% | 0.014% |
| Sodium hydroxide 1M q.s. pH 5.5 | - | - | q.s | q.s | - | q.s |
| Hydrochloric acid 1 M qs pH 5.5 | - | - | q.s | q.s | - | q.s |
| Hydroxypropyl gamma-cyclodextrin | - | 1% | - | 1% | 1% | - |
| Water for Injection (USP) q.s.p | 1 ml q.s. | 1 ml q.s. | 1 ml q.s. | 1 ml q.s. | 1 ml q.s. | 1 ml q.s. |

The pharmaceutical compositions described above were prepared according to conventional state of the art processes.

### Example 2

Pharmaceutical compositions according to Example 1 (F1) conveyed in a plastic bag of PP tri-laminate were evaluated after fabrication, using pH Metro model 744, chromatograph model Prominence and particle counter Model 8000A.

The results are shown in the following table:

**Table 2 - Stability test results**

| Test | Specification | 50 ml | 100 ml | 125 mL |
|---|---|---|---|---|
| Appeara nce | Clear liquid, slightly yellow, free from foreign particles | Approve d | Approved | Approved |
| Volume | Minimum of 100% of the specified value | 51.25 | 101.17 | 126.3 |
| pH | 5,0 to 6,5 | 5.82 | 5.82 | 5.83 |
| Foreign particles | Maximum: 25 particles/ml ≥10µm Minimum: 3 | 5.00 particles/ ml ≥10µm 1.20 | 8.5 particles/ml ≥10µm 0.4 particles/mL | 5.00 particles/m I ≥10pm 0.4 particles/m |
| | particles/mL ≥25µm | particles/ mL ≥25µm | >25µm | L ≥25µm |
| Identific ation | Paracetamol | Positive | Positive | Positive |
| Paracet amol content | % = 95 to 105 | 99.72 | 100.99% | 104.2% |
| Related substan ces | 4-aminophenol <0.5% Another impurity <0.1% Total impurities <1.0% | 0% | 0% | 0% |
| | | 0% | 0% | 0% |
| | | 0% | 0% | 0% |

The technique man will promptly know to evaluate, through the teachings contained in the text and in presented examples, advantages of the invention and propose variations and equivalents alternatives of embodiment, without departing from the scope of the invention as defined by the attached claims.

## Claims

1. STABILIZED PARACETAMOL PHARMACEUTICAL COMPOSITION **characterized in that** it comprises at least one substance which reacts with phenolates derived from paracetamol degradation.

2. Composition according to claim 1, **characterized in that** the substance is selected from at least one of reducing sugars such as glucose, galactose, fructose, mannitol, acid forms of these sugars or their salts such as mannitol, gluconate, glucuronate, glucoheptanoate, galactato, chemical species containing sulfur in an oxidation state less than 6, sodium formaldehyde sulfoxylate, thiourea, sulfite, sodium dithionite, cysteine hydrochloride monohydrate, sodium phosphate dibasic anhydrous, hydroxyethyl, hydroxypropyl gamma-cyclodextrin, sodium acetate trihydrate, or any combination of the previous substances.

3. COMPOSITION according to claim 2, **characterized in that** the substance is selected from mannitol, glucose, fructose, or gluconate in a concentration between 0.2% to 3.5% w/v and/or sodium formaldehyde sulfoxylate, sulfite sodium or sodium dithionite in a concentration between 0.0008% and 0.02% w/v.

4. COMPOSITION according to claim 2, **characterized in that** the substance is mannitol at a concentration between 0.6% to 3.3% w/v.

5. COMPOSITION according to claim 2, **characterized in that** the substance is cysteine hydrochloride monohydrate in a concentration of 0.01% to 1 % w/v, in particular at a concentration of 0.01 % to 0.5% w/v.

6. COMPOSITION according to claim 2, **characterized in that** the substance is anhydrous dibasic sodium phosphate at a concentration between 0.01 % to 1% w/v.

7. COMPOSITION according to claim 2, **characterized by** the fact that the substance is hydroxyethyl starch (HES1) at a concentration between 0.1% to 2% w/v, particularly at a concentration between 0.1% to 1.5% w/v.

8. COMPOSITION according to claim 2, **characterized in that** the substance is hydroxypropyl gamma-cyclodextrin at a concentration between 0.1% to 2% w/v, particularly at a concentration between 0.1% to 1.5% w/v.

9. COMPOSITION according to claim 2, **characterized in that** the substance is sodium acetate trihydrate in a concentration between 0.1% to 1% w/v, particularly at a concentration between 0.1 % to 0.5% w/v.

10. COMPOSITION according to claim 1, **characterized in that** further comprises one or more substances to adjust the pH from 5.5 to 5.7, 1 M solution, selected from sodium hydroxide, glacial acetic acid, hydrochloric acid, citrate sodium dihydrate.

11. COMPOSITION according to claim 10, **characterized in that** sodium citrate dihydrate is used in a concentration from 0.1% to 1% w/v.

12. COMPOSITION according to claim 1, **characterized in that** further comprises one or more stabilizing osmolarity substances, which can be glucose, galactose, fructose or mannitol, to obtain an isotonic solution with osmolarity of 260 mOsm to 320 mOsm, more particularly at a concentration between 0.5% to 5%, particularly 3.3%.

13. PLASTIC BAG **characterized in that** it comprises the pharmaceutical composition according to claim 1.

14. BAG according to claim 13, **characterized in that** it consists of polypropylene tri-laminate.

15. USE OF THE PHARMACEUTICAL COMPOSITION according to claim 1 **characterized in that** being in manufacturing a plastic bag ready for use.

16. USE in accordance with claim 15, **characterized in that** the pharmaceutical composition is administered intravenously.
